# EUROPEAN PATENT SPECIFICATION

(11) **EP 1 402 826 B1**
(45) Date of publication and mention of the grant of the patent: **12.06.2013**
(21) Application number: 03017643.2
(22) Date of filing: 12.08.2003
(51) Int. Cl.: A61F 2/01

(54) **Thrombus capture catheter**
Katheter zum Einfangen von Thromben
Cathéter de capture de thrombus

(30) Priority: 20.08.2002 JP 2002239678
(43) Date of publication of application: 31.03.2004
(73) Proprietor: NIPRO CORPORATION, Kita-ku, Osaka-shi, Osaka-fu, 531-0073 (JP)
(72) Inventor: Isshiki, Takaaki, Setagaya-ku, Tokyo-to (JP); Kataishi, Yuichi, Yokohama-shi, Kanagawa-ken (JP); Miyagawa, Katsuya, Kita-ku, Osaka-shi, Osaka-fu (JP); Kataoka, Hideaki, Kita-ku, Osaka-shi, Osaka-fu (JP)
(74) Representative: Vossius & Partner

(56) References cited:
- WO-A-00/53120
- WO-A1-01/97714
- US-A- 5 876 367
- US-A- 6 152 946
- US-A1- 2002 055 747

## Description

The present invention relates to a thrombus capture catheter and, more particularly, a catheter for capturing fragmentary thrombi (blood clots) pushed out of a diseased site when applying percutaneous transluminal angioplasty to an angiostenosis site such as coronary artery, carotid artery, a venous graft or the like.

Percutaneous transluminal angioplasty such as stent, Plain old Balloon Atherectomy (POBA) has long been applied to an angiostenosis site such as coronary arteries, carotid artery, venous grafting, or the like to expand a narrowed flow path in the blood vessel. This procedure is one of recanalization techniques for securing the blood flow in which the angiostenosis site is expanded by balloon inflation and then kept open by stenting. The angioplasty produces good results.

The above procedure makes it possible to expand the angiostenosis site, but allows the plaques or blood clot to be occasionally liberated in fragments from the wall of the blood vessel during procedure. The fragments flow from the diseased site toward the peripheral blood vessel and cause infarction or "no-reflow", which leads to failure in reperfusion. The fragments of the plaque or clot may cause a serious complication.

For example, when applying the angioplasty to unstable angina or -acute myocardial infarction involved with the blood clot in the coronary artery, the clot may be pushed out of the diseased site by stem placement or balloon inflation. If the clot occludes the blood vessel downstream of the diseased site, the cardiac muscle is prevented from sufficient furnishing of nutrition and oxygen, resulting in myocardial failure such as myocardial necrosis. Similarly, occlusion of blood vessels that run to the brain causes cerebral infarct, resulting in death of the brain tissues. In particularly, arteriosclerosis easily causes constriction of the carotid artery, which may produce insufficiency of the blood flow to the brain, or cerebral infarction due to the blood clot formed at the angiostenosis site. Symptoms of the cerebral infarction include paralysis, palsy, aphasia, consciousness disorder and the like. A patient who has lost its function is limited in complete restoration of the function even if the patient has been subjected to rehabilitative therapies. As a result, the quality of life of the patient would be decline.

Further, there is the need to exercise great care when applying angioplasty to the angiostenosis site of older venous grafting since a great deal of thrombus adheres to the grafting.

The infarction due to thrombus causes serious complications as mentioned above. Thus, the percutaneous transluminal angioplasty needs protection of blood vessels downstream of the diseased site. To this end, there have been developed some catheter devices such as a medical wire (Guard Wire, Trade Name, cf. JP2001-514544A, WO98/39048), and a distal protection device (Filter Wire EX, Trade Name, cf. JP2002-505151A, WO99/44542).

The catheter device specified as the above medical wire comprises a hollow guide wire provided at a distal end thereof with a balloon member. Using this device, the angioplasty is carried out by a method comprising the steps of: locating the distal end of the guide wire through the diseased site; inflating the balloon with an additional device; performing stent placement, Plain Old Balloon Atherectomy (POBA) and the like, and aspirating thrombus with an additional aspirating device. During the procedure, the blood flow in the blood vessel downstream of the diseased site is interrupted, and thus making it possible to prevent outflow of the thrombus pushed out of the diseased site. However, this procedure makes it impossible to keep sufficient flow of the blood to the downstream vessels of the diseased site during procedure. If such an ischemic interval is lengthened, it exerts a great influence on the patient. Further, this procedure requires special devices for inflation and deflation of the balloon, so that it complicates handling of the device and requires a special training of the operator.

The catheter device specified as the above distal protection device comprises a guide wire provided at a distal end thereof with a filter and makes it possible to collect floating emboli in the filter while allowing the blood to flow therethrough to the downstream side of the diseased site. However, the guide wire is biased from the center of the filter, so that the filter may be prevented from uniform contact with the wall of the blood vessel, allowing the thrombus to flow to the downstream side of the diseased site.

For the reasons mentioned above, there is a great demand for development of a device for use in percutaneous transluminal angioplasty including stenting, POBA and the like, which ensures blood flow to the downstream of the diseased site of the blood vessel during procedure, close contact to the wall of the blood vessel for complete collection of thrombus or the like, and operationality of the catheter.

WO 01/97714 relates to a transcatheter embolic protection device having a collapsible filter element.

US 5,876,367 discloses a shunt for maintaining distal blood flow during an arteriotomy procedure. The shunt has a first tubular member for allowing blood flow and a second tubular member for allowing introduction of a blood filter device into within an artery. The second tubular member protrudes outwardly from the first tubular member.

It is therefore an object of the present invention to provide a thrombus capture catheter, which reliably catches fragmentary thrombus (blood clots) and makes it easy to perform percutaneous transluminal angioplasty without stopping the blood flow to peripherals through a diseased site of a blood vessel.

The present invention has been made on the finding that the above object can be achieved by employing, as a thrombus capture member, a wire member comprising plural wires spirally configured to form a configuration swollen in middle portion and tapered to proximal and distal ends. The wire member may be provided with a filter on a distal side including the swollen middle portion thereof.

According to the present invention, there is provided a thrombus capture catheter according to claim 1.

In a preferred embodiment, the thrombus capture member further comprises a filter provided with pores and mounted on a distal side of said crossed wire member including the enlarged portion of said crossed wire member. Combined use of the crossed wire member and the filter makes it possible to capture smaller fragmentary thrombus. The pores of the -filter may have a diameter ranging from 50 to 1000 micrometers, preferably from 50 to 500 micrometers, and more preferably from 100 to 200 micrometers.

Further, the closing member may be provided with a side infusion tube for infusion of heparin to prevent coagulation of the blood.

As will be understood from the above, the thrombus capture catheter according to the present invention enables to capture thrombus reliably and to perform percutaneous transluminal angioplasty with ease without preventing the blood to flow to peripheral blood vessels through a diseased site of the blood vessel.

The invention will be explained below on the basis of the accompanying drawings, which show preferred embodiments of the present invention.
Fig. 1 is a schematic diagram of an illustrative catheter;
Fig. 2 is a schematic diagram illustrating an embodiment of the present invention;
Fig. 3 is a plane view illustrating one embodiment of a thrombus capture member according to the present invention;
Fig. 4 is an enlarged section view of a distal portion of an illustrative catheter including a thrombus capture member in Figs. 2 and 3;
Fig. 5 is an enlarged section view taken along a line X-X in Fig. 3;
Fig. 6 is an enlarged section view illustrating a distal portion of an illustrative catheter including a thrombus capture member;
Fig. 7 is a schematic diagram illustrating percutaneous transluminal angioplasty employing an illustrative thrombus capture catheter;
Fig. 8 is a schematic diagram illustrating percutaneous transluminal angioplasty employing an illustrative thrombus capture catheter;
Fig. 9 is a schematic diagram illustrating percutaneous transluminal angioplasty employing an illustrative thrombus capture catheter ;
Fig. 10 is a schematic diagram illustrating percutaneous transluminal angioplasty employing an illustrative thrombus capture catheter;
Fig. 11 is a schematic diagram illustrating percutaneous transluminal angioplasty employing an illustrative thrombus capture catheter;
Fig. 12 is a schematic diagram illustrating percutaneous transluminal angioplasty employing an illustrative thrombus capture catheter; and
Fig. 13 is an enlarged section view of distal portion illustrating another embodiment of the present invention where the wires are crossed.

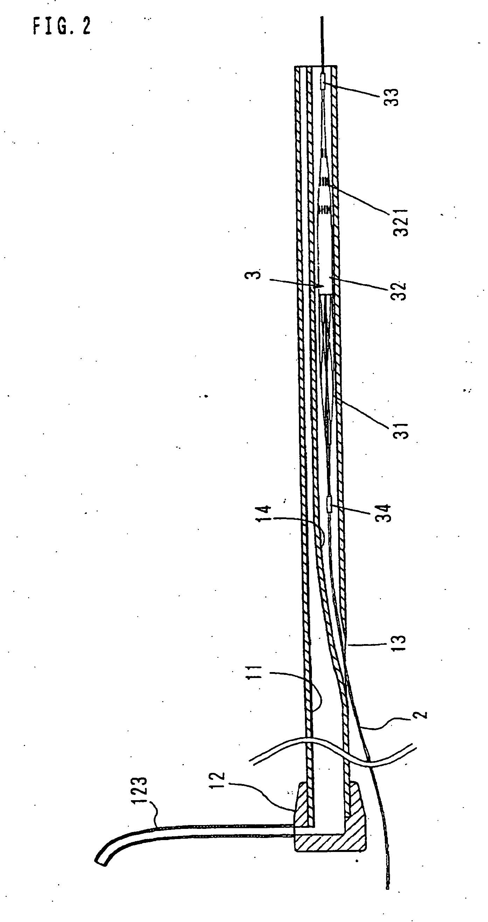

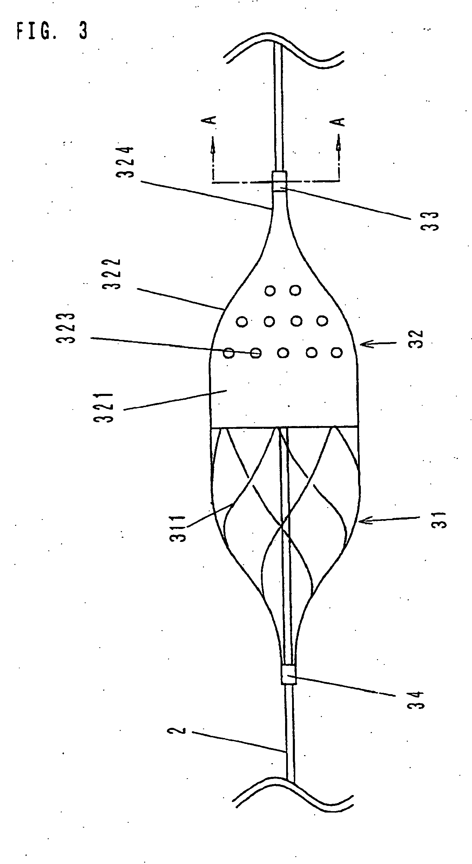

As illustrated in Figs. 2 and 3, a thrombus capture catheter according to the present invention comprises a sheath 1 with a lumen 11 passing therethrough and being closed at a proximal end thereof, a flexible shaft 2 inserted in the lumen 11 of the sheath 1, and a thrombus capture member 3. The thrombus capture member 3 may include a crossed wire member 31 and a filter 32. The crossed wire member 31 comprises plural wires spirally configured and crossed with one another to form a configuration swollen in middle portion and tapered to proximal and distal ends thereof from a swollen middle portion thereof. The crossed wire member 31 is mounted slidably at a distal end thereof on the shaft 2 and fixed at the proximal end thereof to the shaft 2. The crossed wire member 31 is inserted into the sheath 1 and held therein in a constricted condition. By this construction, if the sheath 1 is pulled toward the proximal end, the thrombus capture member 3 is exposed from the sheath 1 and put out.

The sheath 1 is a tubular member made of a plastic material such as polyurethane, polyester, polyethylene, fluorine-containing resins, or the like, and is provided with a lumen 11 extending from the proximal end thereof Lo the distal end thereof. The sheath 1 is provided on the proximal end thereof with a closing member 12 to close a proximal end of the lumen 11.

The closing member 12 is a tubular member 12 made of a plastic material such as polypropylene, ABS (acrylonitrile-butadiene-styrene) copolymer, polyvinyl chloride, polyethylene, polyethylene terephthalate resin, or the like; or a metal such as stainless steel, brass, or the like. The closing member 12 is provided at a distal side thereof with an open end into which the sheath 1 is inserted, while it is closed at a proximal end thereof. In order to allow the shaft 2 to be moved back and forth from outside, it is required to derive the shaft 2 from the sheath 1 to the outside. To this end, the closed proximal end of the closing member 12 may be provided with a through-hole 121 coaxial to the closing member 12, and a hemostatic valve 122 adjacent to the through-hole 121 and in contact with the distal end of the closing member 12. As illustrated in Fig. 2, according to the invention the sheath 1 is provided in a side wall thereof with a side hole 13 allowing the shaft 2 to pass therethrough, and on a distal side thereof with a second lumen 14, which is communicated with the side hole 13 and adapted to accommodate the thrombus capture member 3 therein, to allow the proximal portion of the shaft to pass through the side hole 13 to the outside of the sheath 1.

The closing member 12 may be provided with a side infusion tube 123 for infusion of heparin or the like to keep blood from thrombusting.

The shaft 2 and the thrombus capture member 3 mounted on the distal portion thereof are inserted into and located in the sheath 1. The shaft 2 is a wire-like member made of a flexible metal material such as stainless steel, nickel-titanium alloy, or the like and provides enough flexibility to be deformed to pass through the side hole 13 as shown in Fig. 2. The shaft 2 may be a guide wire used in percutaneous transcatheter angioplasty. The shaft 2 is provided at a distal portion thereof with a thrombus capture member 3 surrounding the distal portion of the shaft 2. The thrombus capture member 3 is fixed at a proximal end thereof to the shaft 2 and at the distal end thereof slidably mounted on the shaft 2.

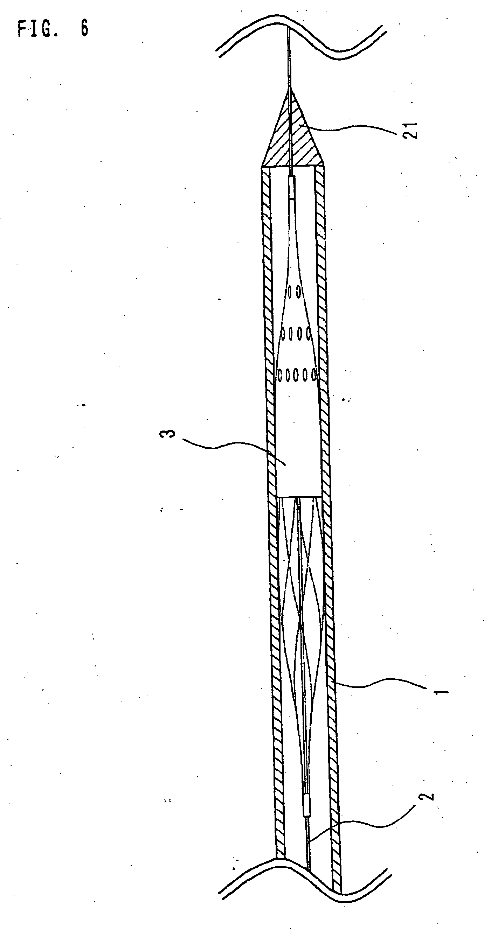

The shaft 2 may be provided at the distal end thereof with a conical member 21 close to the thrombus capture member 3, as shown in Fig. 6, to eliminate difference in surface level between the sheath 1 and the shaft 2. The conical member 21 is a configuration (member) having a proximal end with an outer diameter approximately equal to that of the sheath 1 and being tapered towards a distal end thereof, thereby allowing the thrombus capture catheter to smoothly pass through a diseased site. As a material for the conical member 21, it is preferred to use a biocompatible material same as those used for a filter 32 mentioned later.

The thrombus capture member 3, inserted into the distal portion of the sheath 3 therethrough, comprises a crossed wire member 31 and a filter 32 attached to a distal portion of the crossed wire member 31. The crossed wire member 31 comprises plural wires 311 which are spirally configured and crossed with one another to form a configuration having a swollen middle portion and proximal and distal portions converged from the middle portion to both ends thereof.

The filter 32 is provided with plurality of pores with a diameter ranging from 50 to 1000 micrometers, preferably from 50 to 500 micrometers, and more preferably from 100 to 200 micrometers. If the pore size of the filter 32 is less than 50 micrometers, the filter becomes obstacle to flow of the blood. If the filter 32 has the pores with a diameter exceeding 1000 micrometers, provision of the filter 32 loses its value.

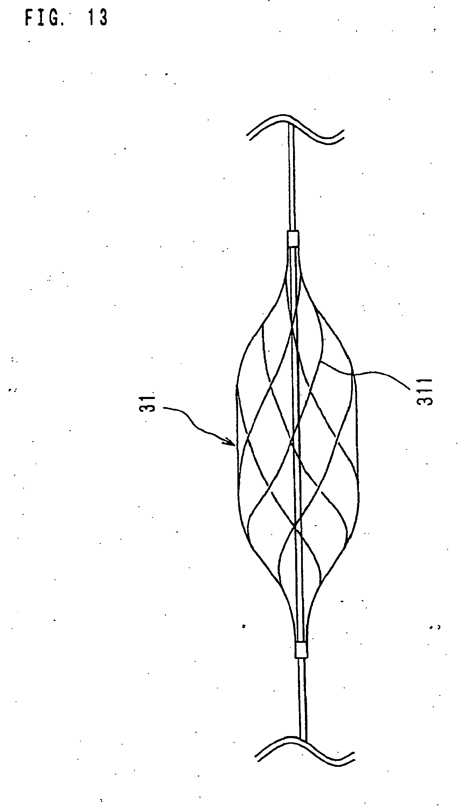

As illustrated in Fig. 13, the thrombus capture member 3 may be comprised of a crossed wire member 31 without any filter. Large thrombus may be captured by a stitched seam of the wire 311. In this case, the crossed wire member 31 may be comprised of 8 to 16 wires, preferably, 8 to 14, and more preferably, 8 to 12 wires.

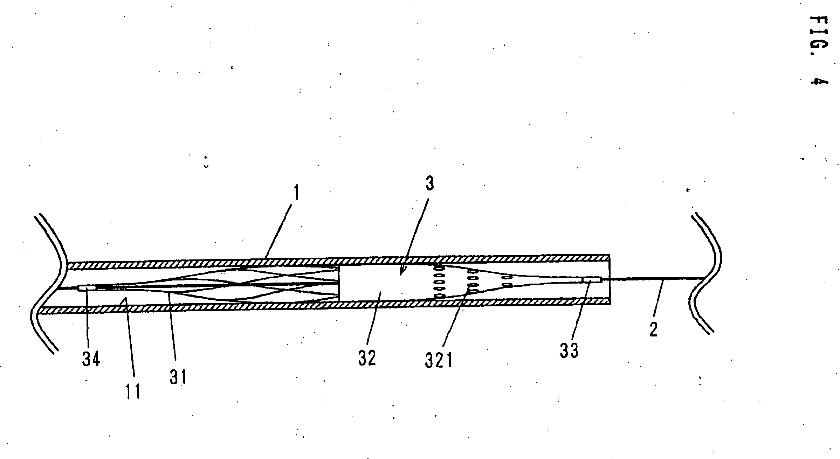

The thrombus capture member 3 is provided at a distal end with a slide ring 33 and at the proximal end with a fixed ring 34. By use of the slidable ring 33 and fixed ring 34, the thrombus capture member 3 is slidably mounted on the shaft 2 by the slidable ring f33 and fixed at the proximal end to the shaft 2 by means of the fixed ring 34. Under the normal condition out of the lumen of the sheath 1, the thrombus capture member 3 takes a swelled configuration as shown in Fig. 3. The thrombus capture member 3 is inserted into and held in the sheath 1 under a shrunk condition as shown in Fig. 4, while it restores to an expanded original state when it is protruded from the sheath by puling the sheath 1 toward the proximal end thereof.

As a material for wire 311, there may be used a shape-memory alloy, which recover its memorized shape at a desired temperature. Generally used alloy is a Ni-Ti alloy, a Cu-Zn-Al alloy, a Cu-Al-Ni alloy, or the like. When the wires 311 are made cf a shape-memory alloy, the thrombus capture member 3, more precisely, the crossed wire member 31 comprised of plural wires 311 spirally formed and crossed with one another, memorizes a shape (an original shape to be restored) as shown in Fig. 3. For application to a diseased site large in thrombus, the thrombus capture member 3 may be formed to have a more long shape so that thrombus are captured as much as possible. Generally, there are prepared a series of thrombus capture members 3 with an outer diameter of 3.0, 3.5, 4.0, 4.5, 5.0, 5.5, 6.0, 6.5, 7.0, 7.5, 8.0 mm. It is preferred to use any thrombus capture member 3 according to the lumen of the diseased site. Of course, the size of the thrombus capture member 3 is never limited to the aforesaid sizes and may take any desired size as occasion demands.

The filter 32 is a funnel-shaped member including with a cylindrical part 321, a constringent part 322 and a constricted part 324 extending from the smaller end of the constringent part 322, as shown in Fig. 3. The constringent part 322 is provided with plural pores 323 with a size, which prevents the thrombus to path through to entrap it in the filter, but allows the body fluid to pass therethrough. The configuration of the filter 32 is not limited-to the aforesaid shape and may be formed into any desired configuration as occasion demands. The filter is made of woven or non-woven fabric consisting of a biocompatible synthetic resin such as polyurethane, polyethylene, polyester, polypropylene, polyamide, polytetrafluoroethylene, polyvinylidene fluoride, or the like material.

The filter 32 is mounted on a distal side of the crossed wire member 31 including a swollen middle portion of the crossed wire member 31 and the cylindrical part 321 is tied to the crossed wire member 31 with a piece of string. The distal end of the constricted part 324 is located adjacent to a slide ring 33 mentioned later. The filter 32 may be formed by dip-molding a resin into a form corresponding to a restored shape of the crossed wire member; and then perforating plural pores with a size that prevents the thrombus to path through to entrap it in the filter but allows the body fluid to pass therethrough. In this case, the resultant filter may be attached to the crossed wire member 31 by mounting it on the expanded open end of the crossed wire member 31.

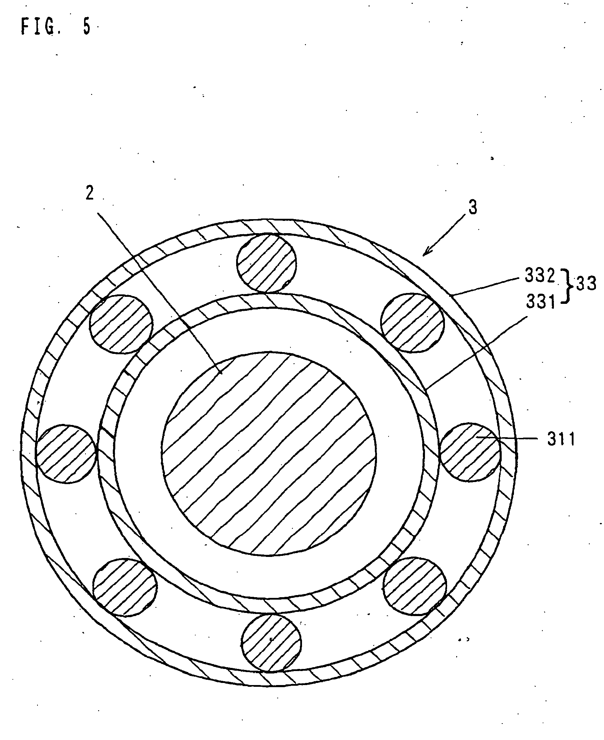

Fig. 5 shows a cross section taken along the line A-A in Fig. 3, illustrating a relationship between a slide ring assembly 33 and a shaft 2. The slide ring assembly 33 includes an inner ring 331 and an outer ring 332, which are made of a metal such as stainless steel or the like. The inner ring 331 is slidably mounted on the shaft 2. The wires 311 are arranged between the inner and outer rings 331 and 332 and fixed thereto by press-fitting or spot welding with a laser welding machine such as CO₂ laser welding machine, YAG laser welding machine, or the like.

The fixed ring assembly 34 has the same structure as that of the slidable ring 33 except for that the inner ring of the fixed ring assembly 34 is fixed to the shaft 2. The fixing of the fixed ring assembly 34 to the shaft 2 may be carried out by press-fitting or spot welding such as CO2 laser welding, YAG laser welding, or the like. Alternatively, the fixed ring. assembly 34 may be fixed to the shaft 2 by providing the shaft 2 with annular ribs (not illustrated in the drawings) close to both proximal and distal ends of the fixed ring assembly 34.

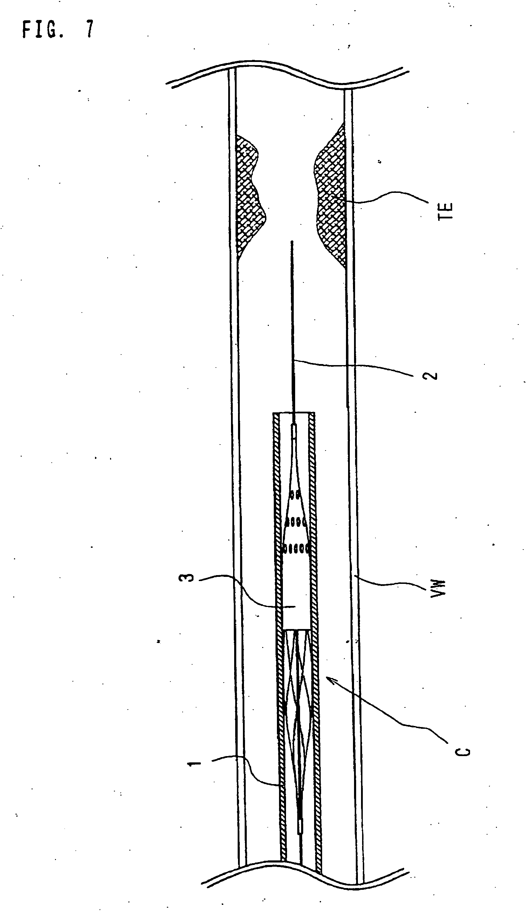

Percutaneous transluminal angioplasty with a thrombus capture catheter will be explained below in reference to Figs. 7 to 12, which are schematic diagrams illustrating steps of a procedure in percutaneous transluminal angioplasty employing a thrombus capture catheter.

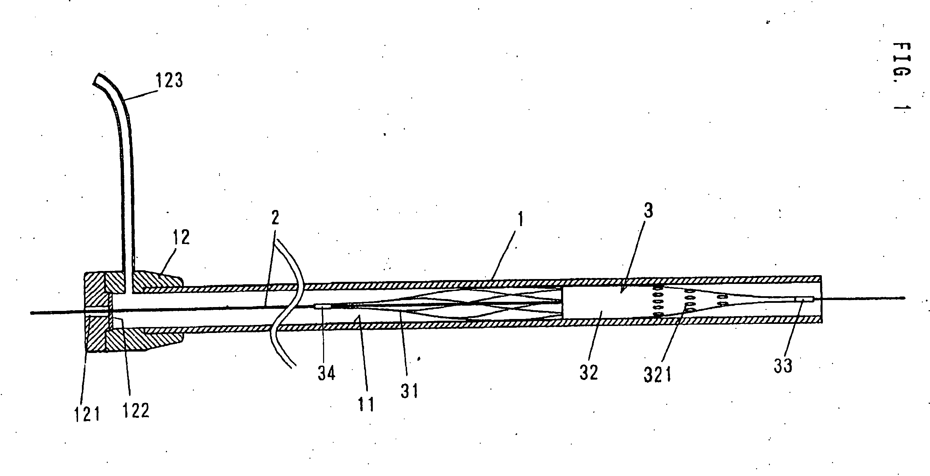

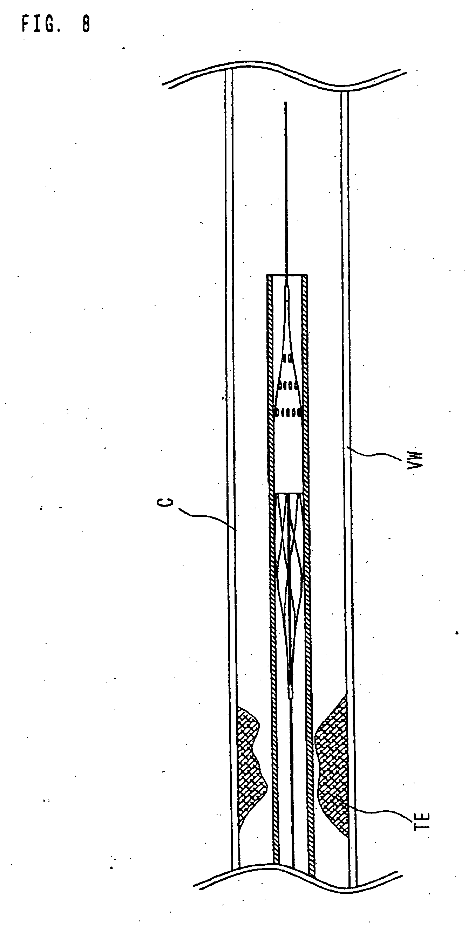

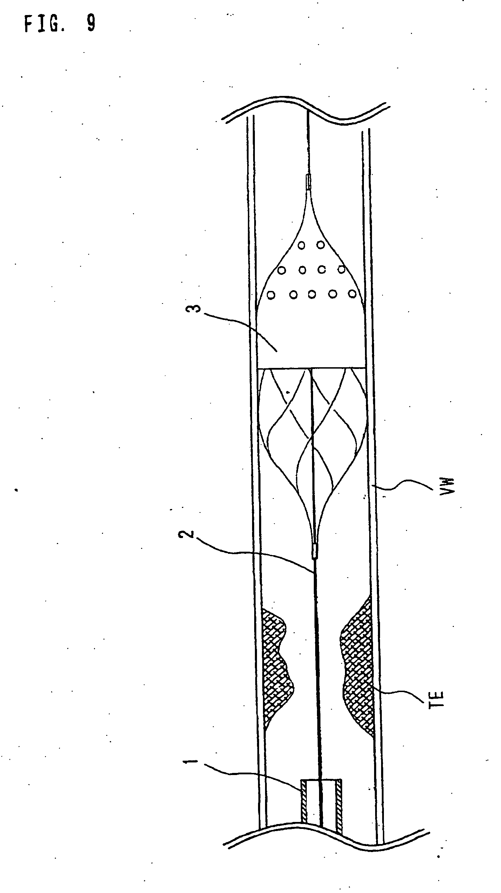

Firstly, the thrombus capture catheter C as shown in Fig. 1 is prepared and then inserted into a guiding catheter (not illustrated in the drawings) previously indwelled near a blood vessel to be treated. The distal end of the shaft 2 is introduced into a vasoconstriction site TE (c.f. Fig. 7), and then the distal end of the sheath 1 in which the thrombus capture member 3 is housed under a shrunk condition is introduced into a distal side of a vasoconstriction site TE (Fig. 8). By pulling the sheath 1 while keeping the shaft 2 under the fixed condition, the thrombus capture member 3 is taken out of the lumen 11 of the sheath 1 and then restored to its original state to make close contact with the vascular wall VW (of. Fig. 9).

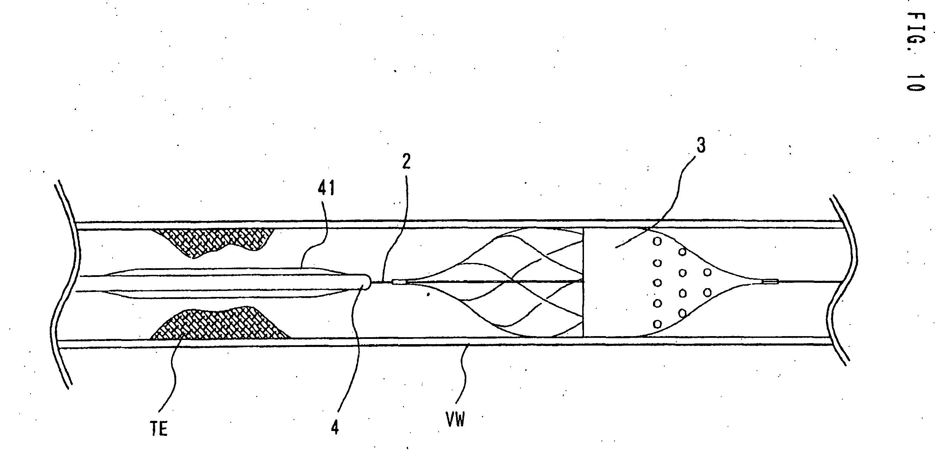

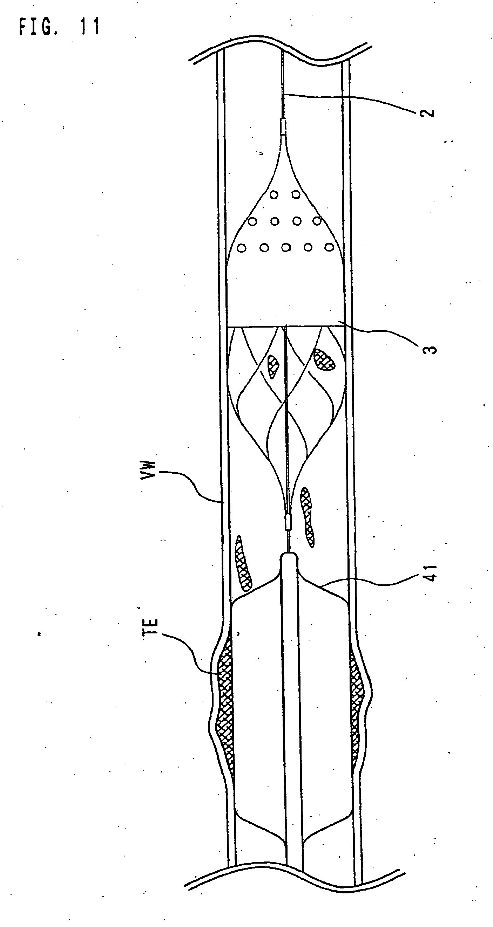

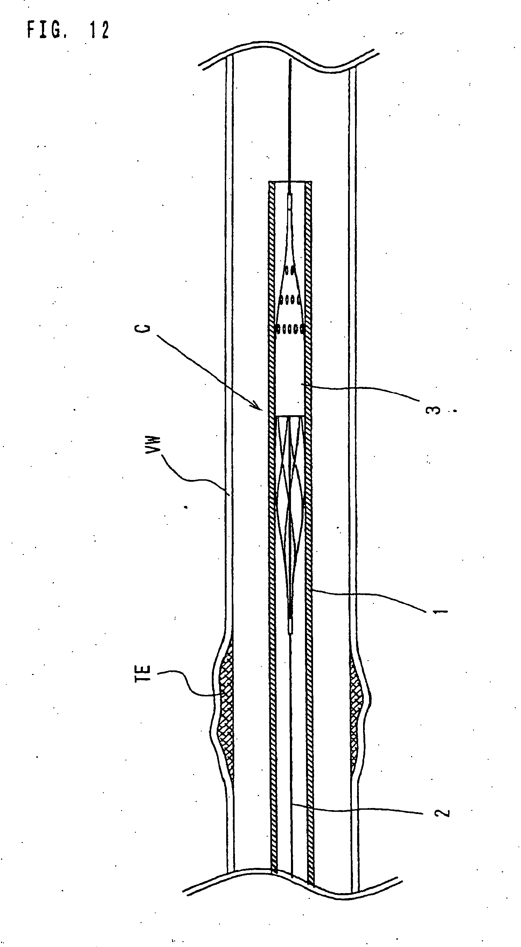

Then, the sheath 1 is withdrawn from the guiding catheter and then a previously prepared balloon catheter is inserted into the vasoconstriction site TE along the shaft 2 (cf. Fig. 10). By expanding the balloon 41, the thrombus is peeled off from the vascular wall VW, liberated into the blood, and then entrapped by the thrombus capture member 3 (cf. Fig. 11). After expansion of the blood vessel, the balloon catheter 4 is withdrawn from the guiding catheter and then the sheath 1 is inserted into the vasoconstriction site TE along the shaft 2. The sheath 1 is then pushed to the distal side of the blood vessel while keeping the shaft 2 under the fixed state, the thrombus capture member 3 is put in the sheath 1 again as shown in Fig. 12. Finally, the thrombus capture catheter C is withdrawn from the blood vessel along with the guiding catheter to complete the Percutaneous transluminal angioplasty.

## Claims

1. A thrombus capture catheter comprising:
a sheath (1) with a lumen (11) passing therethrough from a proximal end thereof to a distal end thereof and being closed at the proximal end thereof by a closing member (12);
a flexible shaft (2) having a proximal end and a distal end and being removably arranged in the lumen (11) of said sheath (1); and
a thrombus capture member (3) provided on the distal end of said shaft (2) and removably arranged in said sheath (1) through the distal end thereof, said thrombus capture member (3) comprising a wire member (31) comprised of plural wires (311) spirally configured to have a configuration swollen in middle portion and tapered to proximal and distal ends thereof, said thrombus capture member (3) being mounted slidably on said shaft (2) at the distal end thereof but fixed to said shaft (2) at the proximal end thereof, said thrombus capture member (3) being held in a contracted state within said sheath (1) and expanded to an original state thereof when protruded from said sheath (1) by pulling said sheath (1) in the direction of the proximal end thereof,
**characterized in that**
said sheath (1) is provided in a side wall close to the distal end thereof with a side hole (13) allowing the shaft (2) to pass therethrough, and with a second lumen (14) communicated with said side wall and allowing said thrombus capture member (3) to pass through, a part of said shaft (2) extending beyond a proximal side of said thrombus capture member (3) being protruded from the sheath (1) through said side hole (13).

2. The thrombus capture catheter according to claim 1, wherein said wire member (31) further comprises a filter (32) provided with pores and mounted on a distal side of said wire member (31) including the enlarged portion of said wire member (31).

3. The thrombus capture catheter according to claim 1 or 2, wherein pores of said filter (32) have a diameter ranging from 50 to 1000 micrometers, preferably from 50 to 500 micrometers, and more preferably from 100 to 200 micrometers.

4. The thrombus capture catheter according to any of claims 1 to 3, wherein said closing member (12) is provided with a side infusion tube (123).

## Patentansprüche

1. Katheter zum Einfangen von Thromben mit:
einer Hülle (1) mit einem Lumen (11), das von ihrem proximalen Ende zu ihrem distalen Ende reicht und an ihrem proximalen Ende durch ein Schließteil (12) verschlossen ist;
einem flexiblen Schaft (2) mit einem proximalen und einem distalen Ende, die in dem Lumen (11) der Hülle (1) entfernbar angeordnet ist; und
einem Thrombuseinfangteil (3), das am distalen Ende des Schafts (2) vorgesehen ist und in der Hülle (1) durch ihr distales Ende reichend entfernbar angeordnet ist, wobei das Thrombuseinfangteil (3) ein Drahtteil (31) aufweist, das aus mehreren spiralförmig konfigurierten Drähten (311) besteht, so dass es eine Konfiguration hat, die im mittleren Abschnitt dick und zu ihrem proximalen und distalen Ende verjüngt ist, wobei das Thrombuseinfangteil (3) an seinem distalen Ende auf dem Schaft (2) gleitfähig angeordnet ist, aber an seinem proximalen Ende an dem Schaft (2) fixiert ist, wobei das Thrombuseinfangteil (3) in einem kontrahierten Zustand in der Hülle (1) gehalten wird und sich zu seinem ursprünglichen Zustand ausdehnt, wenn es durch Ziehen der Hülle (1) in Richtung ihres proximalen Endes aus der Hülle (1) herausgeholt wird,
**dadurch gekennzeichnet, dass**
die Hülle (1) in einer Seitenwand nahe ihrem distalen Ende versehen ist mit einem seitlichen Loch (13), durch das der Schaft (2) hindurch geführt werden kann, und einem zweiten Lumen (14), das mit der Seitenwand in Verbindung steht und durch das das Thrombuseinfangteil (3) hindurch geführt werden kann, wobei ein Teil des Schafts (2), der sich jenseits einer proximalen Seite des Thrombuseinfangteils (3) erstreckt, durch das seitliche Loch (13) aus der Hülle (1) herausgeholt wird.

2. Katheter zum Einfangen von Thromben nach Anspruch 1, wobei das Drahtteil (31) ferner ein Filter (32) aufweist, das mit Poren versehen ist und an einer distalen Seite des Drahtteils (31), einschließlich des vergrößerten Abschnitts des Drahtteils (31), angeordnet ist.

3. Katheter zum Einfangen von Thromben nach Anspruch 1 oder 2, wobei Poren des Filters (32) einen Durchmesser haben, der von 50 bis 1000 Mikrometer, vorzugsweise von 50 bis 500 Mikrometer und besonders bevorzugt von 100 bis 200 Mikrometer reicht.

4. Katheter zum Einfangen von Thromben nach einem der Ansprüche 1 bis 3, wobei das Schließteil (12) mit einer seitlichen Infusionsröhre (123) versehen ist.

## Revendications

1. Cathéter de capture de thrombus comprenant :
une gaine (1) avec une lumière (11) passant à travers cette dernière, de son extrémité proximale à son extrémité distale et étant fermée au niveau de son extrémité proximale par un élément de fermeture (12) ;
une tige souple (2) ayant une extrémité proximale et une extrémité distale et étant agencée de manière amovible dans la lumière (11) de ladite gaine (1) ; et
un élément de capture de thrombus (3) prévu sur l'extrémité distale de ladite tige (2) et agencé de manière amovible dans ladite gaine (1) par son extrémité distale, ledit élément de capture de thrombus (3) comprenant un élément formant fil (31) composé de plusieurs fils (311) configurés en spirale pour avoir une configuration renflée dans la partie centrale et progressivement rétrécie vers ses extrémités proximale et distale, ledit élément de capture de thrombus (3) étant monté de manière coulissante sur ladite tige (2) au niveau de son extrémité distale, mais fixé sur ladite tige (2) au niveau de son extrémité proximale, ledit élément de capture de thrombus (3) étant maintenu dans un état contracté à l'intérieur de ladite gaine (1) et expansé dans son état d'origine, lorsqu'il fait saillie de ladite gaine (1), en tirant ladite gaine (1) dans la direction de son extrémité proximale,
**caractérisé en ce que** :
ladite gaine (1) est prévue dans une paroi latérale à proximité de son extrémité distale avec un trou latéral (13) permettant à la tige (2) de passer à travers ce dernier, et avec une seconde lumière (14) qui communique avec ladite paroi latérale et permettant audit élément de capture de thrombus (3) de passer à travers, une partie de ladite tige (2) s'étendant au-delà d'un côté proximal dudit élément de capture de thrombus (3) qui fait saillie de la gaine (1) par ledit trou latéral (13).

2. Cathéter de capture de thrombus selon la revendication 1, dans lequel ledit élément formant fil (31) comprend en outre un filtre (32) prévu avec des pores et monté sur un côté distal dudit élément formant fil (31) comprenant la partie élargie dudit élément formant fil (31).

3. Cathéter de capture de thrombus selon la revendication 1 ou 2, dans lequel les pores dudit filtre (32) ont un diamètre compris entre 50 et 1000 micromètres, de préférence entre 50 et 500 micromètres, et de manière davantage préférée entre 100 et 200 micromètres.

4. Cathéter de capture de thrombus selon l'une quelconque des revendications 1 à 3, dans lequel ledit élément de fermeture (12) est prévu avec un tube de perfusion latéral (123).
